(19) Europäisches Patentamt / European Patent Office / Office européen des brevets

(11) EP 4 163 360 A1

(12) **EUROPEAN PATENT APPLICATION**
published in accordance with Art. 153(4) EPC

(43) Date of publication:
**12.04.2023 Bulletin 2023/15**

(21) Application number: **21828918.9**

(22) Date of filing: **21.05.2021**

(51) International Patent Classification (IPC):
***C12M 1/34*** *(2006.01)* ***G06T 7/00*** *(2006.01)*
***C12Q 1/04*** *(2006.01)*

(52) Cooperative Patent Classification (CPC):
**C12M 1/34; C12Q 1/04; G06T 7/00**

(86) International application number:
**PCT/JP2021/019472**

(87) International publication number:
**WO 2021/261140 (30.12.2021 Gazette 2021/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.06.2020 JP 2020107143**

(71) Applicant: **Kataoka Corporation**
**Kyoto-shi, Kyoto 601-8203 (JP)**

(72) Inventors:
- **MATSUMOTO Junichi**
  **Kyoto-shi, Kyoto 601-8203 (JP)**
- **MORISHITA Tadao**
  **Kyoto-shi, Kyoto 601-8203 (JP)**
- **TABEI Chiharu**
  **Kyoto-shi, Kyoto 601-8203 (JP)**

(74) Representative: **Seemann & Partner Patentanwälte mbB**
**Raboisen 6**
**20095 Hamburg (DE)**

(54) **CELL TREATMENT DEVICE, LEARNING DEVICE, AND LEARNED MODEL PROPOSAL DEVICE**

(57) Provided is a cell treatment apparatus capable of detecting a target cell using a learned model and treating the detected target cell. The cell treatment apparatus of the present invention is a cell treatment apparatus used for cell treatment, including: an observation unit; a laser irradiation unit; and a control unit. The observation unit is configured to capture an image of a cell that is in a cell culture tool. The laser irradiation unit is configured to apply a laser to an inside of the cell culture tool. The control unit includes a detection section, an irradiation region setting section, and a laser irradiation control section. The detection section is configured to detect, using image data that includes the cell captured by the observation unit and a learned model capable of detecting a target cell or a non-target cell, a target cell or a non-target cell in the image data. The irradiation region setting section is configured to set a region where the target cell is present or a region where the non-target cell is not present as a laser irradiation region to be subjected to laser irradiation by the laser irradiation unit. The laser irradiation control section is configured to apply a laser emitted from the laser irradiation unit to the laser irradiation region in the cell culture tool to treat the target cell.

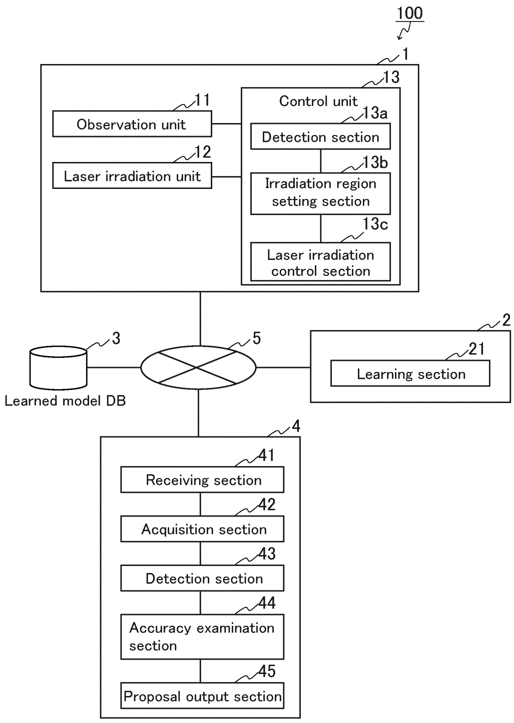


FIG. 1



Processed by Luminess, 75001 PARIS (FR)

## Description

### TECHNICAL FIELD

The present invention relates to a cell treatment apparatus, a learning apparatus, and a proposal apparatus for proposing learned models.

### BACKGROUND ART

In recent years, attempts have been made to cause pluripotent cells such as induced pluripotent stem cells (iPS cells) and embryonic stem cells (ES cells) to differentiate into intended cells, tissues, and the like to utilize them for regenerative medicine and drug discovery.

During maintenance of the pluripotent cells, some of the proliferating pluripotent cells may differentiate into other cells. Further, during differentiation of the pluripotent cells into the intended cells or the like, some of the cells may differentiate into unintended cells.

In such cases, removal of the cells and the like other than the intended cells is currently performed manually. This removing operation, however, requires time and labor since it needs to be carried out, for example, under a microscope. In addition, the quality of cells and the like to be obtained differs greatly depending on the skill level of a person who performs this operation (Patent Literature 1).

### CITATION LIST

### PATENT LITERATURE

Patent Literature 1: JP 2014-509192 A

### SUMMARY OF INVENTION

### TECHNICAL PROBLEM

In light of the foregoing, it is an object of the present invention to provide a cell treatment apparatus capable of detecting target cells using a learned model and treating the detected target cells.

### SOLUTION TO PROBLEM

In order to achieve the above object, the present invention provides a cell treatment apparatus used for cell treatment, including:

an observation unit;
a laser irradiation unit; and
a control unit, wherein
the observation unit is configured to capture an image of a cell that is in a cell culture tool,
the laser irradiation unit is configured to apply a laser to an inside of the cell culture tool,
the control unit includes a detection section, an irradiation region setting section, and a laser irradiation control section,
the detection section is configured to detect, using image data that includes the cell captured by the observation unit and a learned model capable of detecting a target cell or a non-target cell, a target cell or a non-target cell in the image data,
the irradiation region setting section is configured to set a region where the target cell is present or a region where the non-target cell is not present as a laser irradiation region to be subjected to laser irradiation by the laser irradiation unit, and
the laser irradiation control section is configured to apply a laser emitted from the laser irradiation unit to the laser irradiation region in the cell culture tool to treat the target cell.

The present invention also provides a learning apparatus capable of bidirectionally communicating with the cell treatment apparatus of the present invention, including:

a learning section, wherein
the learning section is configured to generate a learned model for detecting a target cell in image data that includes

a cell through machine learning using a set of training data, wherein
the training data comprises
image data captured by the cell treatment apparatus; and
image data that specifies a region where the target cell is present in the image data.

The present invention also provides a proposal apparatus for proposing a learned model used for detection of a target cell, including:

a storage section;
a receiving section;
an acquisition section;
a detection section;
a precision examination section; and
a proposal output section, wherein
the storage section stores a plurality of learned models generated through machine learning using a set of training data composed of: image data that includes a target cell; and image data that specifies a region where the target cell is present in the image data,
the receiving section receives image data that includes a cell,
the acquisition section acquires image data that specifies a region where the target cell is present in the image data received by the receiving section,
the detection section detects, using the image data received by the receiving section and the respective learned models stored in the storage section, the target cell in the image data,
the precision examination section examines a precision of each learned model by comparing the region where the target cell is present, detected using each learned model and the image data that specifies the region where the target cell is present, acquired by the acquisition section, and
when there is a learned model satisfying a predetermined precision level, the proposal output section outputs proposal information that includes information on the learned model satisfying the predetermined precision level.

## ADVANTAGEOUS EFFECTS OF INVENTION

The present invention enables treatment of target cells. Accordingly, the present invention can reduce the cost for cell treatment and can also reduce variations in the quality of cell treatment.

## BRIEF DESCRIPTION OF DRAWINGS

FIG. 1 is a block diagram illustrating an example of the configuration of a cell treatment system including a cell treatment apparatus, learning apparatus, and proposal apparatus according to a first embodiment.
FIG. 2 is a block diagram illustrating an example of the hardware configuration of a control unit included in the cell treatment apparatus of the first embodiment.
FIG. 3 is a block diagram illustrating an example of the hardware configuration of the learning apparatus according to the first embodiment.
FIG. 4 is a block diagram showing an example of the hardware configuration of the proposal apparatus of the first embodiment.
FIG. 5 is a flowchart illustrating an example of a cell treatment method and program of the first embodiment.
FIG. 6 is a flowchart illustrating an example of a learning method and program of the first embodiment.
FIG. 7 is a flowchart illustrating an example of a proposal method and program of the first embodiment.

## DESCRIPTION OF EMBODIMENTS

In the present invention, "cells" mean, for example, isolated cells, or a cell mass (spheroid), tissue, or an organ, each composed of cells. The cells may be, for example, cultured cells or cells isolated from a living body. The cell mass, tissue, or organ may be, for example, a cell mass, cell sheet, tissue, or organ produced from the cells, or may be a cell mass, tissue, or organ isolated from a living body. Cells that adhere in an extracellular matrix (ECM)-dependent manner are preferably used as the cells.
In the present invention, "cell treatment" means treatment of cells, and examples thereof include: removing unnecessary cells by, for example, killing (causing death of) cells or separating cells from a cell culture vessel; selecting

necessary cells; and processing a cell aggregate such as a cell sheet or an organ into a desired shape. Cells to be subjected to the "cell treatment" may be the target cells, the non-target cells, or cells that will differentiate (change) into non-target cells or target cells in the future.

A cell treatment system including the cell treatment apparatus, learning apparatus, and proposal apparatus according to the present invention will be described in detail below with reference to the drawings. It is to be noted, however, that the present invention is not limited by the following description. In FIGs. 1 to 7 to be described below, identical parts are given the same reference numerals, and redundant explanations thereof may be omitted. In the drawings, the structure of each part may be shown in a simplified form as appropriate for the sake of convenience in explanation, and each part may be shown schematically with a dimensional ratio and the like that are different from the actual dimension ratio and the like.

(First Embodiment)

The present embodiment is an example of a cell treatment system including a cell treatment apparatus, learning apparatus, and proposal apparatus according to the present invention. FIG. 1 is a block diagram illustrating a cell treatment system 100 including a cell treatment apparatus 1, learning apparatus 2, learned model database (learned model DB) 3, and proposal apparatus 4 according to the present embodiment. As shown in FIG. 1, the cell treatment system 100 includes the cell treatment apparatus 1, the learning apparatus 2, the learned model DB 3, and the proposal apparatus 4. As shown in FIG. 1, the cell treatment apparatus 1 includes an observation unit 11, a laser irradiation unit 12, and a control unit 13. The control unit 13 includes a detection section 13a, an irradiation region setting section 13b, and a laser irradiation control section 13c. As shown in FIG. 1, the learning apparatus 2 includes a learning section 21. The proposal apparatus 4 includes a receiving section 41, an acquisition section 42, a detection section 43, a precision examination section 44, and a proposal output section 45. As shown in FIG. 1, the cell treatment apparatus 1, the learning apparatus 2, the learned model DB 3, and the proposal apparatus 4 are connectable to each other via a communication network 5. The control unit 13 of the cell treatment apparatus 1, the learning apparatus 2, and the proposal apparatus 4 in the present embodiment may be included in a server or the like in the form of a personal computer or system in which a program of the present invention is installed. Although not shown in FIG. 1, the cell treatment apparatus 1, the learning apparatus 2, the learned model DB 3, and the proposal apparatus 4 are also connectable to an external terminal of a system administrator through the communication network 5, and the system administrator may manage the cell treatment apparatus 1, the learning apparatus 2, the learned model DB 3, and the proposal apparatus 4 via the external terminal. Although the cell treatment system 100 includes one cell treatment apparatus 1 in the present embodiment, two or more cell treatment apparatuses may be included.

The communication network 5 is not limited to particular types of networks, and known networks can be used. The communication network 5 may be either wired or wireless, for example. The communication network 5 may be, for example, an Internet network, the World Wide Web (WWW), a telephone network, a local area network (LAN), or a Wireless Fidelity (Wi-Fi). Preferably, the communication network 5 enables bidirectional communication.

(Configuration of Cell Treatment Apparatus)

The observation unit 11 needs only be capable of capturing images of cells in a cell culture tool. The observation unit 11 may be, for example, an optical observation device. The optical observation device may be, for example, a bright-field microscope, a stereoscopic microscope, a phase-contrast microscope, a differential interference microscope, a polarizing microscope, a fluorescence microscope, a confocal laser microscope, a total internal reflection fluorescence microscope, or a Raman microscope.

The cell culture tool may be, for example, a substrate, dish, plate, or flask (cell culture flask), on/in which cells can be cultured. The size, volume, and material of the cell culture tool, whether an adhesion treatment should be performed, and the like can be determined as appropriate according to the type and amount of cells to be cultured in the cell culture tool. The bottom surface of the cell culture tool may be flat or substantially flat, and may also be a rough surface.

The material of the cell culture tool is not limited to particular materials, and may be, for example, a material that transmits a laser applied by the laser irradiation unit to be described below. Specific examples of such a material include plastic and glass that transmit a laser. Examples of the plastic include polystyrene polymers, acrylic polymers (such as polymethyl methacrylate (PMMA)), polyvinylpyridine polymers (such as poly(4-vinylpyridine) and a 4-vinylpyridine-styrene copolymer), silicone polymers (such as polydimethylsiloxane), polyolefin polymers (such as polyethylene, polypropylene, and polymethylpentene), polyester polymers (such as polyethylene terephthalate (PET) and polyethylene naphthalate (PEN)), polycarbonate polymers, and epoxy polymers.

The cell culture tool has one cell culture region or two or more cell culture regions. In the latter case, it can also be said that the cell culture tool has a plurality of wells, for example.

In the cell culture tool, cells are in direct or indirect contact with, for example, the bottom surface. The "direct contact" refers to the state where, for example, the cells are in contact with the bottom surface of the cell culture tool. The "indirect contact" refers to the state where an interposed layer is present on the cell culture tool and the cells are in contact with the interposed layer.

When the cell culture tool has an interposed layer, the interposed layer may be, for example, a cell base layer containing a cell culture base or a photothermal conversion layer capable of converting light into heat. The interposed layer may include the cell base layer and the photothermal conversion layer, for example. In this case, it is preferable that the cell culture tool has the photothermal conversion layer laminated on its bottom surface and the cell base layer laminated on the photothermal conversion layer.

The cell culture base means, for example, a substance that serves as a scaffold of cells during cell culture. The cell culture base may be, for example, an extracellular matrix (ECM) or a substance having a function as a scaffold for cells. Examples of the extracellular matrix include: elastin; entactin; collagens such as type I collagen, type II collagen, type III collagen, type IV collagen, type V collagen, and type VII collagen; tenascin; fibrillin; fibronectin; laminin; vitronectin; proteoglycans each composed of a sulfated glycosaminoglycan such as chondroitin sulfate, heparan sulfate, keratan sulfate, or dermatan sulfate and a core protein; glucosaminoglycans such as chondroitin sulfate, heparan sulfate, keratan sulfate, dermatan sulfate, and hyaluronic acid; Synthemax® (vitronectin derivative), and Matrigel® (a mixture of laminin, type IV collagen, heparin sulfate proteoglycan, entactin/nidogen, etc.). Of these, laminin is preferable. Examples of the laminin include laminin 111, laminin 121, laminin 211, laminin 213, laminin 222, laminin 311 (laminin 3A11), laminin 332 (laminin 3A32), laminin 321 (laminin 3A21), laminin 3B32, laminin 411, laminin 421, laminin 423, laminin 521, laminin 522, and laminin 523. The three numbers in each laminin indicate, from the first to the last number, the names of the constituent subunits of the $\alpha$ chain, the $\beta$ chain, and the $\gamma$ chain, respectively. As a specific example, laminin 111 is composed of an $\alpha$1 chain, a $\beta$1 chain, and a $\gamma$1 chain. The laminin 3A11 is composed of an $\alpha$3A chain, a $\beta$1 chain, and a $\gamma$1 chain. The cell culture base may contain a peptide fragment of any of the above-described proteins or a fragment of any of the above-described sugar chains. As a specific example, the peptide fragment of any of the above-described proteins may be a fragment of laminin, for example. Examples of the fragment of laminin include fragments of the above-described laminins, and specific examples thereof include laminin 211-E8, laminin 311-E8, laminin 411-E8, and laminin 511-E8. The laminin 211-E8 is composed of fragments of the $\alpha$2 chain, the $\beta$1 chain, and the $\gamma$1 chain of laminin. The laminin 311-E8 is composed of fragments of the $\alpha$3 chain, the $\beta$1 chain, and the $\gamma$1 chain of laminin. The laminin 411-E8 is composed of fragments of the $\alpha$4 chain, the $\beta$1 chain, and the $\gamma$1 chain of laminin. The laminin 511-E8 is composed of, for example, fragments of the $\alpha$5, $\beta$1, and $\gamma$1 chains of laminin.

The cell culture base can be denatured indirectly by irradiating the photothermal conversion layer with light (laser), as will be described below. Specifically, the applied light is converted into heat, and the structure of the cell culture base is changed by the thus-generated thermal energy, thereby causing the indirect denaturation. In other words, the cell culture base is denatured by heat generated through the above-described light irradiation.

The cell culture tool includes one or more cell base layers.

The cell base layer may contain other components in addition to the cell culture base. Examples of the other components include buffers, salts, growth factors (cell growth factors), cytokines, and hormones.

The photothermal conversion layer is a layer capable of converting light into heat. The photothermal conversion layer contains, for example, molecules capable of converting light into heat (photothermal conversion molecules). Preferably, the photothermal conversion molecules are composed of, for example, a polymer (macromolecules) containing a dye structure (chromophore) that absorbs a wavelength of light L by the laser irradiation unit 12 to be described below. Preferably, the photothermal conversion molecules can be easily coated onto the cell culture tool. Examples of the dye structure that absorbs light L include derivatives of organic compounds, such as azobenzene, diarylethene, spiropyran, spirooxazine, fulgide, leuco dyes, indigo, carotinoid (such as carotene), flavonoid (such as anthocyanin), and quinoid (such as anthraquinone). Examples of the skeleton constituting the polymer include acrylic polymers, polystyrene polymers, polyolefin polymers, polyvinyl acetate and polyvinyl chloride, polyolefin polymers, polycarbonate polymers, and epoxy polymers. As a specific example, the photothermal conversion molecule may be, for example, poly[methylmethacrylate-co-(disperse yellow-7-methacrylate)] ($(C_5H_8O_2)_m(C_{23}H_{20}N_4O_2)_n$) represented by the following formula (1). In the following formula (1), as the structure of azobenzene in the polymer, not only unsubstituted azobenzene but also any of various structures modified with a nitro group, an amino group, a methyl group, or the like may be employed. In the following formula (1), m and n each represent a mole percentage. The sum of m and n is, for example, 100 mol%. m and n may be the same or different from each other, for example. The photothermal conversion layer may contain, for example, one type of photothermal conversion molecules or two or more types of photothermal conversion molecules.

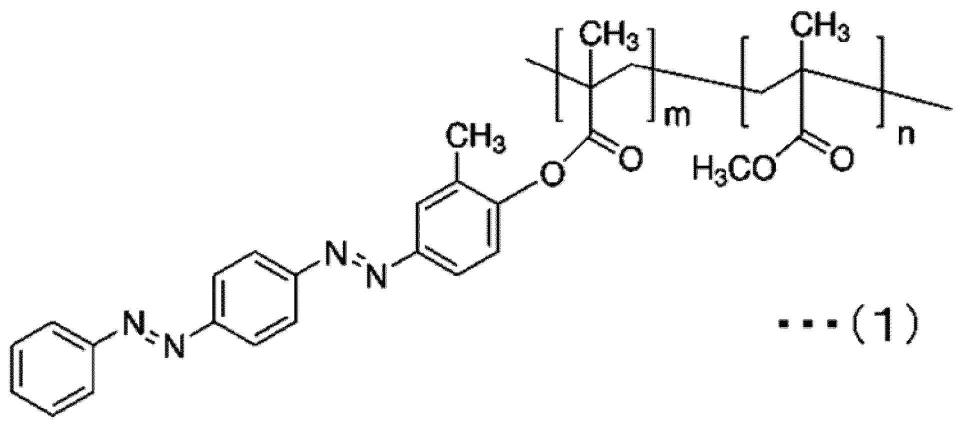

···(1)

The cell culture tool includes one or more photothermal conversion layers. In the cell culture tool, the photothermal conversion layer may be arranged so as to be in contact with the cell base layer or so as not to be in contact with the cell base layer. In the latter case, the photothermal conversion layer and the cell base layer may be thermally connected to each other. Specifically, a heat conductive layer for transferring heat generated in the photothermal conversion layer to the cell base layer is formed between the photothermal conversion layer and the cell base layer. The heat conductive layer contains molecules with high thermal conductivity, such as molecules of a metal, for example.

When the cell culture tool has the cell base layer, the cell base layer may have a region to which cells can adhere and a region where adhesion of cells is inhibited. As to the structure and the production method of the cell culture tool, reference can be made to, for example, the description in WO 2020/071332, which is incorporated herein as part of the present specification.

The laser irradiation unit 12 needs only be capable of applying a laser to the inside of the cell culture tool, and may be, for example, a laser irradiation device. The laser irradiation device includes, for example, a laser source, an optical fiber, and a laser emission section. In this case, the control unit 13 is connected to the laser irradiation unit 12. More specifically, the control unit 13 is connected to the laser source and the laser emission section of the laser irradiation unit 12. The laser irradiation device may guide light using a light guide unit such as a mirror or a micro-electro-mechanical system (MEMS), instead of the optical fiber.

The laser emission section may include, for example, a galvanometer mirror and an fθ lens.

The laser source is a device that emits a continuous-wave laser or a pulsed laser, for example. The laser source may emit, for example, a high-frequency laser that has a long pulse width and approximates to a continuous wave. The output power of a laser emitted by the laser source is not limited to particular values, and can be determined as appropriate according to, for example, the absorption wavelength of the photothermal conversion molecules in the above-described photothermal conversion layer. The wavelength of a laser emitted by the laser source is not limited to particular values, and the laser may be, for example, a laser with a wavelength of 405 nm, 450 nm, 520 nm, 532 nm, or 808 nm, such as a visible-light laser or an infrared laser. As a specific example, the laser source may be a continuous-wave diode laser having a maximum output power of 5 W and a wavelength in the vicinity of 405 nm.

Next, FIG. 2 shows an exemplary block diagram illustrating the hardware configuration of the control unit 13 of the cell treatment apparatus 1. The control unit 13 of the cell treatment apparatus 1 includes, for example, a central processing unit (CPU) 131, a memory 132, a bus 133, a storage device 134, an input device 136, a display 137, a communication device 138, an input-output (I/O) interface 139, etc. The respective sections of the control unit 13 are connected to each other via their respective interfaces (I/F) using the bus 133.

The CPU 131 operates in cooperation with other components under the control of a controller (such as a system controller or an I/O controller) or the like and is responsible for overall control of the control unit 13, for example. In the control unit 13, the CPU 131 executes a program 135 of the present invention and other programs, and reads and writes various types of information, for example. Specifically, for example, the CPU 131 functions as the detection section 13a, the irradiation region setting section 13b, and the laser irradiation control section 13c. Although the control unit 13 includes the CPU as an arithmetic unit, the control unit 13 may include another arithmetic unit such as a graphics processing unit (GPU) or an accelerated processing unit (APU), or may further include such an arithmetic unit in combination with the CPU. The CPU 131 functions, for example, as respective sections other than storage sections in the learning apparatus 2 and the proposal apparatus 4.

Examples of the memory 132 include a main memory. The main memory is also referred to as a main storage device. When the CPU 131 executes processing, the memory 132 reads various operation programs, including a program 135 of the present invention, stored in the storage device 134 (auxiliary storage device) to be described below, for example. Then, the CPU 131 reads out data from the memory 132, decodes the data, and executes the programs. The

main memory is a random-access memory (RAM), for example. Examples of the memory 132 further include a read-only memory (ROM).

The bus 133 can also be connected to the observation unit 11 and the laser irradiation unit 12. The control unit 13 can be connected to the observation unit 11 and the laser irradiation unit 12 via, for example, the I/O interface 139 connected to the bus 133.

The bus 133 can also be connected to, for example, external equipment such as the learning apparatus 2, the learned model DB 3, and the proposal apparatus 4. Examples of the external equipment include an external storage device (such as an external database) and a printer. The control unit 13 can be connected to the communication network 5 via the communication device 138 connected to the bus 133, and can also be connected to the external equipment through the communication network 5. The cell treatment apparatus 1 also can be connected to the learning apparatus 2, the learned model DB 3, and the proposal apparatus 4 via the communication device 138 of the control unit 13 and the communication network 5.

The storage device 134 is also referred to as, for example, a so-called "auxiliary storage device", in contrast to the main memory (main storage device). As described above, the operation programs including the program 135 of the present invention are stored in the storage device 134. The storage device 134 includes, for example, a storage medium and a drive for reading from and writing on the storage medium. The storage medium is not limited to particular types of storage media. The storage medium may be, for example, either a built-in or external storage medium, and examples thereof include a hard disk (HD), a floppy® disk (FD), CD-ROM, CD-R, CD-RW, MO, DVD, a flash memory, and a memory card. The drive is not limited to particular types of drives. The storage device 134 may be, for example, a hard disk drive (HDD) in which the storage medium and the drive are integrated.

The control unit 13 further includes the input device 136 and the display 137, for example. Examples of the input device 136 include: pointing devices such as a touch panel, a trackpad, and a mouse; a keyboard; image capturing means such as a camera and a scanner; card readers such as an IC card reader and a magnetic card reader; and voice input means such as a microphone. Examples of the display 137 include display devices such as an LED display device and a liquid crystal display. Although the input device 136 and the display 137 are provided as separate components in the present embodiment, the input device 136 and the display 137 may be configured integrally as a single component such as a touch panel display.

(Configuration of Learning Apparatus)

Next, FIG. 3 shows an exemplary block diagram illustrating the hardware configuration of the learning apparatus 2. The learning apparatus 2 includes, for example, a central processing unit (CPU) 201, a memory 202, a bus 203, a storage device 204, an input device 206, a display 207, a communication device 208, and an input-output (I/O) interface 209. The respective sections of the learning apparatus 2 are connected to each other via their respective interfaces (I/F) using the bus 203. The above descriptions regarding the respective sections of the control unit 13 also apply to the corresponding sections of the learning apparatus 2.

In the present embodiment, the learned model DB 3 is a database server that stores a plurality of learned models (cell detection models) capable of detecting target cells or non-target cells, as will be described below. The above description regarding the hardware configuration of the learning apparatus 2 also applies to the hardware configuration of the learned model DB 3. The learned model DB 3 may store one learned model or two or more learned models, and the latter is preferable. As the number of learned models stored in the learned model DB 3 increases, a more suitable learned model for the target cells can be proposed by, for example, the proposal apparatus 4 to be described below in the cell treatment system 100 of the present embodiment. It should be noted that, in the present invention, the learned models may be stored in, for example, the storage device 204 of the learning apparatus 2 and/or a storage device 404 of the proposal apparatus 4.

(Configuration of Proposal Apparatus)

Next, FIG. 4 shows an exemplary block diagram illustrating the hardware configuration of the proposal apparatus 4. The proposal apparatus 4 includes, for example, a central processing unit (CPU) 401, a memory 402, a bus 403, a storage device 404, an input device 406, a display 407, a communication device 408, and an input-output (I/O) interface 409. The respective sections of the proposal apparatus 4 are connected to each other via their respective interfaces (I/F) using the bus 403. The above descriptions regarding the respective sections of the control unit 13 also apply to the corresponding sections of the proposal apparatus 4.

(Processing by Cell Treatment Apparatus)

Next, an example of processing performed by each of the apparatuses included in the cell treatment system

100 of the present embodiment will be described with reference to an exemplary case where the cell treatment apparatus 1 treats cells and an exemplary case where processing is performed based on image data that includes an image of target cells captured by the cell treatment apparatus 1.

First, an exemplary case where the cell treatment apparatus 1 treats cells will be described with reference to the flowchart of FIG. 5.

First, prior to treatment by the cell treatment apparatus 1, the user of the cell treatment apparatus 1 inputs a culture condition(s) and/or an image capturing condition(s) of cells in the cell culture tool using the input device 136 of the cell treatment apparatus 1. When an identifier (such as a QR code® or a bar code) associated with data on the culture condition(s) and/or the image capturing condition(s) of the cell culture tool is printed on the cell culture tool, the cell treatment apparatus 1 may identify the identifier printed on the cell culture tool and acquire the data associated with the identifier from a storage device such as a database. When the cell treatment apparatus 1 has an auto-focus function or the like, the cell treatment apparatus 1 may acquire some or all image capturing conditions.

Examples of the culture condition include a cell line name, a cell type, a cell origin, the number of cell passages, a cell seeding density at the start of culture, a culture medium, the number of days used for cell culture, the type of a cell culture vessel, the type of an extracellular matrix, an operator's name, an operator's qualification, and the number of years of operator's work experience. The number of culture conditions may be one, or two or more, for example.

Examples of the image capturing condition include the type of an image sensor, the sensitivity of the image sensor an exposure time, an aperture value, a lens magnification, the type of a light source, the quantity of light, an illumination time, and an observation method. Examples of the type of the image sensor include: the type of image sensor defined by light detectable by the device, such as an image sensor for monochrome image capturing or an image sensor for color image capturing; the number of pixels in the image sensor; and the type of a sensor, such as a charge coupled device (CCD) or a complementary metal oxide semiconductor (CMOS). The sensitivity of the image sensor is, for example, the gain or the ISO sensitivity. Examples of the type of the light source include a light emitting diode (LED) and a halogen lamp. The observation method means an observation method employed by the observation unit 11, and examples thereof include a dark-field observation method, a phase contrast observation method, a differential interference observation method, a polarization observation method, a fluorescence observation method, a relief contrast observation method, and a dispersion observation method.

Next, processing by the cell treatment apparatus 1 is started. First, the observation unit 11 of the cell treatment apparatus 1 captures an image of the inside of the cell culture tool to acquire an image that includes cells (S11). The observation unit 11 captures an image of part or the whole of the inside of the cell culture tool. The image capturing position by the observation unit 11 may be, for example, a position specified in advance or a position specified by the user. The observation unit 11 captures one or more images, for example. The observation unit 11 preferably captures an image in such a manner that the cells in the cell culture tool are included in the captured image, but may capture an image of a region that does not include the cells.

Then, the observation unit 11 associates the data of the captured image (target image) with the culture condition(s) and the image capturing condition(s) and stores the data in the memory 132 or the storage device 134. The observation unit 11 may store the target image data in association with information on the identification number of the cell treatment apparatus 1, such as the manufacturer's serial number, and the like.

In the case where the cell treatment apparatus 1 generates a new learned model from the target image or receives a proposal of a learned model suitable for the target image as will be described below, the cell treatment apparatus 1 may transmit the target image data and the information such as the condition(s) associated with the target image data to the learning apparatus 2 or the proposal apparatus 4 from the communication device 138 of the control unit 13 through the communication network 5 or may store the target image data and the information such as the condition(s) associated with the target image data in a database server. In this case, the cell treatment apparatus 1 may transmit, for example, image data that specifies a region where the target cells are present in the target image data, in association with the target image data. The region where the target cells are present is specified by, for example, acquiring data inputted by the user of the cell treatment apparatus 1.

Next, the control unit 13 sets a region to be subjected to treatment by the laser irradiation unit 12 based on the target image data and performs laser irradiation (S12 to S14).

First, the detection section 13a detects, using the target image data captured by the observation unit 11 and a learned model capable of detecting target cells or non-target cells, target cells or non-target cells in the target image data (S12). The learned model may be, for example, a learned model stored in the cell treatment apparatus 1 or a learned model stored outside the cell treatment apparatus 1. In the former case, the detection section 13a utilizes, for example, a learned model stored in the storage device 134. In the latter case, the detection section 13a acquires a learned model stored in the learned model DB 3 and utilizes the acquired learned model. The method for producing the learned model will be described below. The detection of cells can also be referred to as, for example, identification of cells, discrimination of cells, extraction of cells, estimation of cells, or specification of cells.

The detection section 13a may utilize one or more learned models. In the case where the detection section

13a utilizes a plurality of learned models, the detection section 13a detects target cells or non-target cells in the target image data using the respective learned models and then detects regions where the target cells or non-target cells overlap one another in the target image data detected by the respective learned models, the sum of such regions, or part of such regions as a region where the target cells or non-target cells are present, for example.

The target cells and the non-target cells can be set as appropriate according to the intended use of the cells by the user. The target cells or non-target cells may be: pluripotent cells such as induced pluripotent stem (iPS) cells and embryonic stem (ES) cells; cells of the nervous system, including neuronal cells (such as dopamine neuronal cells), neural stem cells, astrocytes, and glial cells; cells of the visual system, such as corneal cells, retinal cells, and photoreceptor cells; cells of the cardiac muscle, such as myocardial cells; hematopoietic cells and immune cells, such as platelets, T cells, B cells, NK cells, NKT cells, erythrocytes, and hematopoietic stem cells; cells of the digestive system, such as liver cells; cells of the endocrine system, such as pancreatic beta cells and cells of the pituitary gland; cells of the kidney renal and the urinary system, such as nephron progenitor cells; and cells of the locomotor system, such as cartilages and skeletal muscles. The target cells and non-target cells may be cells derived from different origins, such as cells derived from a healthy subject and cells derived from a diseased patient.

Next, the irradiation region setting section 13b sets a region where the target cells are present as a laser irradiation region to be subjected to laser irradiation by the laser irradiation unit 12 (S13). When the target cells are detected by the detection section 13a, the irradiation region setting section 13b sets a region where the target cells are detected, i.e., a region where the target cells are present, as the laser irradiation region. On the other hand, when the non-target cells are detected by the detection section 13a, the irradiation region setting section 13b sets a region where the non-target cells are not detected, i.e., a region where the target cells are present, as the laser irradiation region. The laser irradiation region can be set by, for example, setting coordinates (XY coordinates or XYZ coordinates) on the bottom surface of the cell culture tool and specifying the coordinates on the front surface coordinates or a region on the plane coordinates.

The laser irradiation region may be set such that, for example, the target cells can be treated by applying a laser beam emitted from the laser irradiation unit 12 to the laser irradiation region. As a specific example, a region including the target cells may be set as the laser irradiation region. In the case where the cell culture tool has the photothermal conversion layer, a region corresponding to the region including the target cells, specifically, a corresponding region of the photothermal conversion layer, present immediately below the region including the target cells may be set as the laser irradiation region.

Next, the laser irradiation control section 13c applies a laser emitted from the laser irradiation unit 12 to the laser irradiation region in the cell culture tool to treat the target cells (S14). Specifically, the laser irradiation control section 13c applies a laser emitted from the laser irradiation unit 12 to the laser irradiation region in the cell culture tool to treat the target cells by, for example, controlling the irradiation position of the laser from the laser irradiation unit 12 and the ON/OFF state of the laser emission.

The cell treatment apparatus 1 then finishes the processing.

In the present embodiment, the cell treatment apparatus 1 uses the laser irradiation region set by the irradiation region setting section 13b as it is. However, the present invention is not limited thereto and may be configured so as to enable adjustment of the laser irradiation region. In this case, the control unit 13 of the cell treatment apparatus 1 includes an irradiation region adjustment section for adjusting the position of the boundary of the laser irradiation region based on region adjustment information set in advance. The region adjustment information is information for adjusting the position of the boundary of the laser irradiation region to reduce or enlarge the laser irradiation region. The region adjustment information is, for example, inputted by the user of the cell treatment apparatus 1 in advance. The region adjustment information includes, for example, information that specifies whether the laser irradiation region should be enlarged or reduced and information that specifies the distance by which the boundary of the laser irradiation region is moved in a normal direction to the boundary. In this case, the irradiation region adjustment section moves the position of the boundary of the laser irradiation region in the normal direction by the specified distance based on the region adjustment information, thereby enlarging or reducing the laser irradiation region. Then, the laser irradiation control section 13c applies a laser emitted from the laser irradiation unit 12 to the thus-adjusted laser irradiation region in the cell culture tool to treat the target cells.

(Processing by Learning Apparatus)

Next, an exemplary case where the learning apparatus 2 generates a learned model will be described with reference to the flowchart of FIG. 6.

First, prior to processing by the learning apparatus 2, the cell treatment apparatus 1 acquires target image data used for learning. In the present embodiment, the cell treatment apparatus 1 including the control unit 13 is used to acquire the target image data. However, the present invention is not limited thereto, and the cell treatment apparatus need only include the observation unit 11 and the laser irradiation unit 12, for the purpose of acquiring target image data

used for learning.

The cell treatment apparatus 1 can acquire target image data in the above-described manner. Thereafter, the cell treatment apparatus 1 transmits the target image data and the information such as the condition(s) associated with the target image data to the learning apparatus 2 through the communication network 5.

Next, the learning apparatus 2 generates a learned model from the target image data and stores the learned model (S21 to S23).

First, the communication device 208 (receiving section) of the learning apparatus 2 receives the target image data captured by the cell treatment apparatus 1 and the information such as the condition(s) associated with the target image data (S21). In the case where the cell treatment apparatus 1 stores the target image data and the information such as the condition(s) associated with the target image data in the database server, the learning apparatus 2 may, for example, request the target image data and the information such as the condition(s) associated with the target image to the database server and the receiving section may receive the target image data and the information such as the condition(s) associated with the target image data, transmitted from the database server. The receiving section may receive one target image data or two or more sets of target image data, and the latter is preferable.

The learning apparatus 2 may utilize, for example, target image data captured by one cell treatment apparatus 1 or target image data captured by a plurality of cell treatment apparatuses 1. In the latter case, the learning apparatus 2 is connected to the plurality of cell treatment apparatuses 1 in a bidirectionally communicable manner.

Next, the learning section 21 generates a learned model using the target image data (S22). Specifically, the learning section 21 generates a learned model through machine learning using a region where the target cells are present and a region where the non-target cells are present in the target image data either as positive and negative examples or as negative and positive examples, respectively. First, the learning section 21 determines whether there is image data that specifies the region where the target cells are present in the target image data.

If Yes, i.e., if the target image data includes image data that does not specify the region where the target cells are present (data captured by the cell treatment apparatus 1) and image data that specifies the region where the target cells are present, the learning section 21 generates a learned model through machine learning using a set of training data composed of the image data that does not specify the region where the target cells are present and the image data that specifies the region where the target cells are present.

On the other hand, if No, i.e., if the target image data does not include image data that specifies the region where the target cells are present, the learning section 21 acquires, based on the image data that does not specify the region where the target cells are present, image data that specifies the region where the target cells are present, prior to the generation of the learned model. The image data that specifies the region where the target cells are present can be acquired by, for example, acquiring data inputted by the user of the learning apparatus 2. Then, the learning section 21 generates a learned model through machine learning using a set of training data composed of the image data that does not specify the region where the target cells are present and the image data that specifies the region where the target cells are present.

Learning by the learning section 21 can be performed through machine learning. The machine learning is preferably machine learning using a neural network, for example. The machine learning using a neural network is, for example, machine learning using: a convolutional neural network (CNN); a fully convolutional network such as U-Net or HED; a generative adversarial network (GAN); or an extreme learning machine (ELM). Since the weight of a discriminator, the filter factor, the offset, and the like, for example, are calculated in the above-described machine learning, the machine learning may utilize logistic regression processing. In learning by the learning section 21, one or more machine learning methods are used. When a plurality of machine learning methods are used in learning by the learning section 21, the learning section 21 may generate learned models using the respective machine learning methods, and thereafter, may examine the precision of each of the learned models and select learned models satisfying the criteria set in advance. In this case, the learning section 21 examines the precision of each learned model in the same manner as a precision examination section 44 and a proposal output section 45 of the proposal apparatus 4 to be described below. The learning section 21 may further improve the precision of the learned models generated by the learning section 21 by, for example, manually or automatically adjusting the hyperparameters for the learned models. Also, the learning section 21 may further perform, for example, reinforcement learning for the learned models generated by the learning section 21.

The present embodiment is directed to an example where the learning section 21 newly generates learned models. However, in the case where learned models capable of detecting the target cells are stored in the learned model DB 3, relearning may be performed using the learned models and the target image data etc. received by the receiving section. In this case, the receiving section receives the learned models capable of detecting the target cells transmitted from the learned model DB 3. Further, the learning section 21 may perform model compression for the obtained learned models via distillation or the like.

The learning apparatus 2 then stores the obtained learned model (S23). The learned model may be stored, for example, in the memory 202 or the storage device 204 of the learning apparatus 2 or in a database server provided outside the learning apparatus 2, such as the learned model DB 3. In the present embodiment, the learning apparatus

2 stores the learned model in association with the image capturing condition(s) and/or the culture condition(s). In the cell treatment system 100 of the present embodiment, this enables proposal of a learned model with higher precision when, for example, the proposal apparatus 4 to be described below proposes a learned model. It is to be noted, however, that the present invention is not limited thereto, and the learning apparatus 2 may store the learned model without associating neither the image capturing condition(s) nor the culture condition(s) with the learned model.

The learning apparatus 2 then finishes the processing.

The learning apparatus 2 of the present embodiment may include a validation section that validates, based on the obtained learned model and the training data, the precision of the learned model. As to the validation performed by the validation section, reference can be made to, for example, descriptions regarding the precision validation section 44 and the proposal output section 45 of the proposal apparatus 4 to be described below. When the learning apparatus 2 includes the validation section, the learned model may be generated using part of the image data received by the receiving section, and the validation section may perform validation using the rest of the image data received by the receiving section. When the validation by the validation section shows that the learned model obtained by the learning section 21 does not satisfy a certain precision level, relearning may be performed. As to the determination of the precision, reference can be made to, for example, descriptions regarding the proposal output section 45 of the proposal apparatus 4 to be described below.

(Processing by Proposal Apparatus)

Next, an exemplary case where the proposal apparatus 4 proposes a learned model will be described with reference to the flowchart of FIG. 7.

First, prior to processing by the proposal apparatus 4, the cell treatment apparatus 1 acquires target image data used for proposal of a learned model. In the present embodiment, the cell treatment apparatus 1 including the control unit 13 is used to acquire the target image data. However, the present invention is not limited thereto, and the cell treatment apparatus need only include the observation unit 11 and the laser irradiation unit 12, for the purpose of acquiring target image data used for learning.

The cell treatment apparatus 1 can acquire target image data in the above-described manner. Then, the cell treatment apparatus 1 transmits the target image data and the information such as the condition(s) associated with the target image data to the proposal apparatus 4 through the communication network 5.

Next, the proposal apparatus 4 extracts the learned model from the target image data and proposes the extracted learned model (S41 to S45).

First, the receiving section 41 of the proposal apparatus 4 receives the target image data captured by the cell treatment apparatus 1 and the information such as the condition(s) associated with the target image data (S41). In the case where the cell treatment apparatus 1 stores the target image data and the information such as the condition(s) associated with the target image data in a database server, the proposal apparatus 4 may, for example, request the target image data and the information such as the condition(s) associated with the target image to the database server and the receiving section 41 may receive the target image data and the information such as the condition(s) associated with the target image data, transmitted from the database server. The receiving section 41 may receive one target image data or two or more sets of target image data, and the latter is preferable.

Next, the acquisition section 42 acquires image data that specifies a region where the target cells are present in the target image data received by the receiving section 41 (S42). When the image data that specifies the region where the target cells are present is associated with the target image data received by the receiving section 41, the acquisition section 42 acquires the associated image data that specifies the region where the target cells are present. On the other hand, when the image data that specifies the region where the target cells are present is not associated with the target image data received by the receiving section 41, the acquisition section 42 acquires, for example, image data that specifies the region where the target cells are present, inputted by the user of the proposal apparatus 4.

Next, the detection section 43 detects, using the target image data received by the receiving section 41 and each learned model stored in the learned model DB 3, target cells in the target image data (S43).

Next, the precision examination section 44 examines the precision of each learned model by comparing a region where the target cells are present, detected using each learned model and the image data that specifies the region where the target cells are present, acquired by the acquisition section 42 (S44). The precision examination section 44 may further examine the precision of each learned model by using a region where the target cells are not present, detected using each learned model and image data that specifies the region where the target cells are not present, acquired by the acquisition section 42. Examples of the precision include the accuracy, recall, true positive rate, false positive rate, false negative rate, true negative rate, negative predictive value, and specificity.

As a specific example, the precision can be examined by, for example, comparing a region where the target cells are present, detected using each learned model (target region) or a region where the target cells are not present, detected using each learned model (non-target region) and the region where the target cells are present in the image

data acquired by the acquisition section 42 (present region) or the region where the target cells are not present in the image data acquired by the acquisition section 42 (absent region). As a specific example, for each target image data acquired by the acquisition section 42, the precision examination section 44 examines which of the classifications in Table 1 below applies based on whether the respective pixels in the target region and the non-target region overlap the pixels in the present region and the absent region, and counts the number of applied classifications. Then, the precision examination section 44 examines the precision based on the thus-obtained counts, for example. The respective types of precision can be calculated as per the following equations.

$$\text{Accuracy} = (TP + TN)/(TP + TN + FP + FN)$$

$$\text{Recall} = TP/(TP + FN)$$

$$\text{Goodness of Fit} = TP/(TP + FP)$$

$$\text{Specificity} = TN(FP + TN)$$

$$\text{Negative Predictive Value} = TN/(FN + TN)$$

[Table 1]

| | Detection result using learned model | |
|---|---|---|
| | Target region | Non-target region |
| Present region | True positive (TP) | True negative (TN) |
| Absent region | False positive (FP) | False negative (FN) |

If there is a learned model satisfying the predetermined precision level, the proposal output section 45 outputs proposal information that includes information on the learned model satisfying the predetermined precision level (S45). Specifically, the proposal output section 45 first determines whether the precision of each learned model determined by the precision examination section 44 satisfies the predetermined precision level. If Yes, i.e., if there is a learned model satisfying the predetermined precision level, the proposal output section 45 extracts information on the learned model satisfying the predetermined precision level. Then, the proposal output section 45 outputs the extracted information on the learned model to the cell treatment apparatus 1 that transmitted the target image data. The proposal information may include image data for which the extracted learned model was used to detect the target cells. On the other hand, if No, i.e., if there is no learned model satisfying the predetermined precision level, the proposal output section 45 does not extract information on the learned model satisfying the predetermined precision level. Then, the proposal output section 45 outputs that there is no learned model satisfying the required condition to the cell treatment apparatus 1 that transmitted the target image data.

The predetermined precision level can be set as appropriate according to, for example, treatment to be performed by the cell treatment apparatus 1. When the treatment is to kill the target cells, it is preferable to set the predetermined precision level using accuracy and recall as indices, because it can prevent some of the target cells to be killed from remaining untreated. In this case, the proposal output section 45 extracts a learned model determined by the precision examination section 44 as having an accuracy value equal to or greater than a predetermined value and a recall value equal to or greater than a predetermined value, and then outputs information on the extracted learned model as proposal information via the communication device 408 to the cell treatment apparatus 1 that transmitted the target image data.

On the other hand, in the case where there is no learned model satisfying the predetermined precision level, the proposal output section 45 may output, to the cell treatment apparatus 1 that transmitted the target image data, that there is no learned model satisfying the condition or proposal information that proposes the generation of a new learned model.

The proposal apparatus 4 then finishes the processing.

In the proposal apparatus 4 of the present embodiment, the information such as the image capturing condition(s)

and/or the culture condition(s) associated with the target image data and the information such as the image capturing condition(s) and/or the culture condition(s) associated with the learned model are not utilized for the proposal of the learned model. However, the present invention is not limited thereto, and the image capturing condition(s) and the culture condition(s) may also be utilized. In this case, for example, the proposal apparatus 4 of the present embodiment includes a model extraction section for extracting the learned model based on the image capturing condition(s) and/or the culture condition(s) associated with the target image data.

The model extraction section may perform extraction with respect to, for example, learned models used in the detection section 43 or learned models extracted by the proposal output section 45. When the model extraction section performs extraction with respect to learned models used in the detection part 43, the model extraction section may extract a learned model suitable for the image data received by the receiving section 41 based on the image capturing condition(s) and/or the culture condition(s) associated with the image data received by the receiving section 41 and the image capturing condition(s) and/or the culture condition(s) associated with each learned model stored in the learned model DB 3. The extraction can be performed by checking the image capturing condition(s) and the culture condition(s) associated with the target image data against the image capturing condition(s) and the culture condition(s) associated with each learned model, and extracting a learned model with some or all of the image capturing condition(s) and the culture condition(s) associated with the target image data match the image capturing condition(s) and the culture condition(s) associated with each learned model. In this case, the detection section 43 performs detection in the same manner as described above, using the learned model extracted by the model extraction section.

The present embodiment enables treatment of target cells. Accordingly, for example, the present embodiment can reduce the cost for cell treatment and can also reduce variations in the quality of cell treatment. Further, according the present embodiment, a new learned model or an improved learned model can be generated using image data captured by the cell treatment apparatus 1 and can be accumulated in a database. Since the cell treatment apparatus 1 can capture images of any cells, the cell treatment system 100 of the present embodiment can generate and accumulate learned models for any target cells. Thus, according to the present embodiment, any target cells can be treated. Moreover, the present embodiment can reduce the cost for cell treatment and can reduce variations in the quality of cell treatment for any target cells.

(Second Embodiment)

The present embodiment is directed to a program that can execute the above-described cell treatment method, learning method, or proposal method on a computer. Alternatively, the program of the present embodiment may be recorded on, for example, a computer-readable recording medium. The recording medium is, for example, a non-transitory computer-readable storage medium. The recording medium is not limited to particular types of recording media, and may be, for example, a random-access memory (RAM), a read-only memory (ROM), a hard disk (HD), a hard disk (HD), an optical disk, or a floppy® disk (FD).

While the present invention has been described above with reference to exemplary embodiments, the present invention is by no means limited to the above embodiments. Various changes and modifications that may become apparent to those skilled in the art may be made in the configuration and specifics of the present invention without departing from the scope of the present invention.

This application is based upon and claims the benefit of priority from Japanese Patent Application No. 2020-107143 filed on June 22, 2020, the entire disclosure of which is incorporated herein by reference.

<Supplementary Notes>

The whole or part of the exemplary embodiments and examples disclosed above can be described as, but not limited to, the following supplementary notes.

(Supplementary Note 1)

A cell treatment apparatus used for cell treatment, including:

an observation unit;
a laser irradiation unit; and
a control unit, wherein
the observation unit is configured to of capture an image of a cell that is in a cell culture tool,
the laser irradiation unit is configured to of apply a laser to an inside of the cell culture tool,
the control unit includes a detection section, an irradiation region setting section, and a laser irradiation control section,
the detection section is configured to detect, using image data that includes the cell captured by the observation

unit and a learned model capable of detecting a target cell or a non-target cell, a target cell or a non-target cell in the image data,
the irradiation region setting section is configured to set a region where the target cell is present or a region where the non-target cell is not present as a laser irradiation region to be subjected to laser irradiation by the laser irradiation unit, and
the laser irradiation control section is configured to apply a laser emitted from the laser irradiation unit to the laser irradiation region in the cell culture tool to treat the target cell.

(Supplementary Note 2)

The cell treatment apparatus according to Supplementary Note 1, wherein

the control unit further includes an irradiation region adjustment section,
the irradiation region adjustment section is configured to adjust a position of a boundary of the laser irradiation region based on region adjustment information set in advance,
the region adjustment information is information for adjusting the position of the boundary of the laser irradiation region to reduce or enlarge the laser irradiation region, and
the laser irradiation control section is configured to apply a laser emitted from the laser irradiation unit to the thus-adjusted laser irradiation region in the cell culture tool to treat the target cell.

(Supplementary Note 3)

The cell treatment apparatus according to Supplementary Note 2, wherein

the region adjustment information includes information that specifies whether the laser irradiation region should be enlarged or reduced and information that specifies a distance by which the boundary of the laser irradiation region is moved in a normal direction to the boundary, and
the irradiation region adjustment section is configured to move the position of the boundary of the laser irradiation region in the normal direction by the specified distance based on the region adjustment information, thereby enlarging or reducing the laser irradiation region.

(Supplementary Note 4)

The cell treatment apparatus according to any one of Supplementary Notes 1 to 3, wherein the learned model is generated through machine learning using a set of training data, wherein

the training data comprises
image data that includes a target cell; and
image data that specifies a region where the target cell is present in the image data.

(Supplementary Note 5)

The cell treatment apparatus according to any one of Supplementary Notes 1 to 4, wherein the treatment is to kill the cell.

(Supplementary Note 6)

A learning apparatus capable of bidirectionally communicating with the cell treatment apparatus according to any one of Supplementary Notes 1 to 5, the learning apparatus including:

a learning section, wherein
the learning section is configure to generate a learned model for detecting a target cell in image data that includes a cell through machine learning using a set of training data, wherein
the training data comprises
image data captured by the cell treatment apparatus; and
image data that specifies a region where the target cell is present in the image data.

(Supplementary Note 7)

The learning apparatus according to Supplementary Note 6, further including a receiving section, wherein the receiving section is configured to receive the image data captured by the cell treatment apparatus.

(Supplementary Note 8)

The learning apparatus according to Supplementary Note 6 or 7, wherein

the image data captured by the cell treatment apparatus is associated with an image capturing condition of the image date captured by the cell treatment apparatus and/or a culture condition of the cell, and
the learning section is configured to associate the generated learned model with the image capturing condition and/or the culture condition.

(Supplementary Note 9)

The learning apparatus according to Supplementary Note 8, wherein
the culture condition is at least one condition selected from the group consisting of a cell line name, a cell type, the number of cell passages, a cell seeding density at the start of culture, a culture medium, the number of days used for cell culture, a type of a cell culture vessel, a type of an extracellular matrix, an operator's name, an operator's qualification, and the number of years of operator's work experience.

(Supplementary Note 10)

The learning apparatus according to Supplementary Note 8 or 9, wherein
the image capturing condition is at least one condition selected from the group consisting of a type of an image sensor, a sensitivity of the image sensor, an exposure time, an aperture value, a lens magnification, a type of a light source, a quantity of light, an illumination time, and an observation method.

(Supplementary Note 11)

The learning apparatus according to any one of Supplementary Notes 6 to 10, further including an acquisition section, wherein
the acquisition section is configured to acquire the image data that specifies the region where the target cell is present in the image data captured by the cell treatment apparatus.

(Supplementary Note 12)

The learning apparatus according to any one of Supplementary Notes 6 to 11, further including a validation section, wherein
the validation section is configured to validate a precision of the learned model based on the obtained learned model and the training data.

(Supplementary Note 13)

The learning apparatus according to any one of Supplementary Notes 6 to 12, further including a storage section, wherein
the storage section is configured to store the learned model.

(Supplementary Note 14)

The learning apparatus according to any one of Supplementary Notes 6 to 13, capable of bidirectionally communicating with a plurality of the cell treatment apparatuses.

(Supplementary Note 15)

A proposal apparatus for proposing a learned model used for detection of a target cell, the proposal apparatus including:

a storage section;
a receiving section;
an acquisition section;
a detection section;
a precision examination section; and
a proposal output section, wherein
the storage section stores a plurality of learned models generated through machine learning using a set of training data composed of: image data that includes a target cell; and image data that specifies a region where the target cell is present in the image data,
the receiving section receives image data that includes a cell,
the acquisition section acquires image data that specifies a region where the target cell is present in the image data received by the receiving section,
the detection section detects, using the image data received by the receiving section and the respective learned models stored in the storage section, the target cell in the image data,
the precision examination section examines a precision of each learned model by comparing the region where the target cell is present, detected using each learned model and the image data that specifies the region where the target cell is present, acquired by the acquisition section, and
when there is a learned model satisfying a predetermined precision level, the proposal output section outputs proposal information that includes information on the learned model satisfying the predetermined precision level.

(Supplementary Note 16)

The proposal apparatus according to Supplementary Note 15, wherein, when there is no learned model satisfying the predetermined precision level, the proposal output section outputs proposal information that proposes generation of a new learned model.

(Supplementary Note 17)

The proposal apparatus according to Supplementary Note 15 or 16, further including a model extraction section, wherein

each learned model is associated with an image capturing condition of the image data that includes the target cell and is used in the generation of the learned model and/or a culture condition of the cell,
the image data received by the receiving section is associated with the image capturing condition of the image data and/or the culture condition of the cell,
the model extraction section extracts a learned model suitable for the image data received by the receiving section based on the image capturing condition and/or the culture condition associated with the image data received by the receiving section and the image capturing condition and/or the culture condition associated with each learned model, and
the detection section detects, using the image data received by the receiving section and the extracted learned model, the target cell in the image data.

(Supplementary Note 18)

A cell treatment method performed by a cell treatment apparatus used for cell treatment,

the cell treatment apparatus including:

an observation unit; and
a laser irradiation unit, wherein
the observation unit is capable of capturing an image of a cell that is in a cell culture tool, and
the laser irradiation unit is capable of applying a laser to an inside of the cell culture tool,

the cell treatment method including:

a detection step;
an irradiation region setting step; and
a laser irradiation control step, wherein

in the detection step, using image data that includes the cell captured by the observation unit and a learned model capable of detecting a target cell or a non-target cell, a target cell or a non-target cell in the image data is detected,
in the irradiation region setting step, a region where the target cell is present or a region where the non-target cell is not present is set as a laser irradiation region to be subjected to laser irradiation by the laser irradiation unit, and
in the laser irradiation control step, a laser emitted from the laser irradiation unit is applied to the laser irradiation region in the cell culture tool to treat the target cell.

(Supplementary Note 19)

The cell treatment method according to Supplementary Note 18, further including an irradiation region adjustment step, wherein

in the irradiation region adjustment step,
a position of a boundary of the laser irradiation region is adjusted based on region adjustment information set in advance,
the region adjustment information is information for adjusting the position of the boundary of the laser irradiation region to reduce or enlarge the laser irradiation region, and
in the laser irradiation control step, a laser emitted from the laser irradiation unit is applied to the thus-adjusted laser irradiation region in the cell culture tool to treat the target cell.

(Supplementary Note 20)

The cell treatment method according to Supplementary Note 19, wherein

the region adjustment information includes information that specifies whether the laser irradiation region should be enlarged or reduced and information that specifies a distance by which the boundary of the laser irradiation region is moved in a normal direction to the boundary, and
in the irradiation region adjustment step, the position of the boundary of the laser irradiation region is moved in the normal direction by the specified distance based on the region adjustment information, whereby the laser irradiation region is enlarged or reduced.

(Supplementary Note 21)

The cell treatment method according to any one of Supplementary Notes 18 to 20, wherein the learned model is generated through machine learning using a set of training data composed of: image data that includes a target cell; and image data that specifies a region where the target cell is present in the image data.

(Supplementary Note 22)

The cell treatment method according to any one of Supplementary Notes 18 to 21, wherein the treatment is to kill the cell.

(Supplementary Note 23)

A learning method performed by a learning apparatus, the learning method including:

a learning step, wherein
in the learning step, a learned model for detecting a target cell in image data that includes a cell is generated through machine learning using a set of training data composed of: image data captured by the cell treatment apparatus; and image data that specifies a region where the target cell is present in the image data.

(Supplementary Note 24)

The learning method according to Supplementary Note 23, further including a receiving step, wherein
in the receiving step, the image data captured by the cell treatment apparatus is received.

(Supplementary Note 25)

The learning method according to Supplementary Note 23 or 24, wherein

the image data captured by the cell treatment apparatus is associated with an image capturing condition of the image date captured by the cell treatment apparatus and/or a culture condition of the cell, and
in the learning step, the generated learned model is associated with the image capturing condition and/or the culture condition.

(Supplementary Note 26)

The learning method according to Supplementary Note 25, wherein the culture condition is at least one condition selected from the group consisting of a cell line name, a cell type, the number of cell passages, a cell seeding density at the start of culture, a culture medium, the number of days used for cell culture, a type of a cell culture vessel, a type of an extracellular matrix, an operator's name, an operator's qualification, and the number of years of operator's work experience.

(Supplementary Note 27)

The learning method according to Supplementary Note 25 or 26, wherein the image capturing condition is at least one condition selected from the group consisting of a type of an image sensor, a sensitivity of the image sensor, an exposure time, an aperture value, a lens magnification, a type of a light source, a quantity of light, an illumination time, and an observation method.

(Supplementary Note 28)

The learning method according to any one of Supplementary Notes 23 to 27, further including an acquisition step, wherein
in the acquisition step, the image data that specifies the region where the target cell is present in the image data captured by the cell treatment apparatus is acquired.

(Supplementary Note 29)

The learning method according to any one of Supplementary Notes 23 to 28, further including a validation step, wherein
in the validation step, based on the obtained learned model and the training data, a precision of the learned model is validated.

(Supplementary Note 30)

The learning method according to any one of Supplementary Notes 23 to 29, further including a storing step, wherein
in the storing step, the learned model is stored.

(Supplementary Note 31)

The learning method according to any one of Supplementary Notes 23 to 30, wherein a plurality of the cell treatment apparatuses is used.

(Supplementary Note 32)

A learned-model proposal method performed by a proposal apparatus for proposing a learned model used for detection of a target cell, the proposal method including:

a receiving step;
an acquisition step;
a detection step;
a precision examination step; and

a proposal output step, wherein
in the receiving step, image data that includes a cell is received,
in the acquisition step, image data that specifies a region where the target cell is present in the image data received in the receiving step is acquired,
in the detection step, using the image data received in the receiving step and each learned model stored in the storage section, the target cell in the image data is detected,
the storage section stores a plurality of learned models generated through machine learning using a set of training data composed of: the image data that includes the target cell; and the image data that specifies the region where the target cell is present in the image data,
in the precision examination step, a precision of each learned model is examined by comparing the region where the target cell is present, detected using each learned model stored in the storage section and the image data that specifies the region where the target cell is present, acquired in the acquisition step, and
in the proposal output step, when there is a learned model satisfying a predetermined precision level, proposal information that includes information on the learned model satisfying the predetermined precision level is outputted.

(Supplementary Note 33)

The proposal method according to Supplementary Note 32, wherein, in the proposal output step, when there is no learned model satisfying the predetermined precision level, proposal information that proposes generation of a new learned model is outputted.

(Supplementary Note 34)

The proposal method according to Supplementary Note 32 or 33, further including a model extraction step, wherein

each learned model is associated with an image capturing condition of the image data that includes the target cell and is used in the generation of the learned model and/or a culture condition of the cell,
the image data received in the receiving step is associated with the image capturing condition of the image data and/or the culture condition of the cell,
in the model extraction step, a learned model suitable for the image data received in the receiving step is extracted based on the image capturing condition and/or the culture condition associated with the image data received in the receiving step and the image capturing condition and/or the culture condition associated with each learned model, and
in the detection step, using the image data received in the receiving step and the extracted learned model, the target cell in the image data is detected.

INDUSTRIAL APPLICABILITY

The present invention enables treatment of target cells.
Accordingly, the present invention can reduce the cost for cell treatment and can also reduce variations in the quality of cell treatment. Therefore, the present invention is very useful, for example, in the fields of regenerative medicine and drug discovery.

## Claims

**1.** A cell treatment apparatus used for cell treatment, comprising:

an observation unit;
a laser irradiation unit; and
a control unit, wherein
the observation unit is configured to capture an image of a cell that is in a cell culture tool,
the laser irradiation unit is configured to apply a laser to an inside of the cell culture tool,
the control unit comprises a detection section, an irradiation region setting section, and a laser irradiation control section,
the detection section is configured to detect, using image data that comprises the cell captured by the observation unit and a learned model capable of detecting a target cell or a non-target cell, a target cell or a non-target cell in the image data,

the irradiation region setting section is configured to set a region where the target cell is present or a region where the non-target cell is not present as a laser irradiation region to be subjected to laser irradiation by the laser irradiation unit, and
the laser irradiation control section is configured to apply a laser emitted from the laser irradiation unit to the laser irradiation region in the cell culture tool to treat the target cell.

**2.** The cell treatment apparatus according to claim 1, wherein

the control unit further comprises an irradiation region adjustment section,
the irradiation region adjustment section is configured to adjust a position of a boundary of the laser irradiation region based on region adjustment information set in advance, wherein
the region adjustment information is information for adjusting the position of the boundary of the laser irradiation region to reduce or enlarge the laser irradiation region, and
the laser irradiation control section is configured to apply a laser emitted from the laser irradiation unit to the thus-adjusted laser irradiation region in the cell culture tool to treat the target cell.

**3.** The cell treatment apparatus according to claim 2, wherein

the region adjustment information comprises information that specifies whether the laser irradiation region should be enlarged or reduced and information that specifies a distance by which the boundary of the laser irradiation region is moved in a normal direction to the boundary, and
the irradiation region adjustment section is configured to move the position of the boundary of the laser irradiation region in the normal direction by the specified distance based on the region adjustment information, thereby enlarging or reducing the laser irradiation region.

**4.** The cell treatment apparatus according to any one of claims 1 to 3, wherein the learned model is generated through machine learning using a set of training data, wherein

the training data comprises
image data that comprises a target cell; and
image data that specifies a region where the target cell is present in the image data.

**5.** The cell treatment apparatus according to any one of claims 1 to 4, wherein
the treatment is to kill the cell.

**6.** A learning apparatus capable of bidirectionally communicating with the cell treatment apparatus according to any one of claims 1 to 5, the learning apparatus comprising:

a learning section, wherein
the learning section is configured to generate a learned model for detecting a target cell in image data that comprises a cell through machine learning using a set of training data, wherein
the training data comprises
image data captured by the cell treatment apparatus; and
image data that specifies a region where the target cell is present in the image data.

**7.** The learning apparatus according to claim 6, further comprising a receiving section, wherein
the receiving section is configured receive the image data captured by the cell treatment apparatus.

**8.** The learning apparatus according to claim 6 or 7, wherein

the image data captured by the cell treatment apparatus is associated with an image capturing condition of the image date captured by the cell treatment apparatus and/or a culture condition of the cell, and
the learning section is configured to associate the generated learned model with the image capturing condition and/or the culture condition.

**9.** The learning apparatus according to claim 8, wherein
the culture condition is at least one condition selected from the group consisting of a cell line name, a cell type, the number of cell passages, a cell seeding density at the start of culture, a culture medium, the number of days used

for cell culture, a type of a cell culture vessel, a type of an extracellular matrix, an operator's name, an operator's qualification, and the number of years of operator's work experience.

**10.** The learning apparatus according to claim 8 or 9, wherein the image capturing condition is at least one condition selected from the group consisting of a type of an image sensor, a sensitivity of the image sensor, an exposure time, an aperture value, a lens magnification, a type of a light source, a quantity of light, an illumination time, and an observation method.

**11.** The learning apparatus according to any one of claims 6 to 10, further comprising an acquisition section, wherein the acquisition section is configured to acquire the image data that specifies the region where the target cell is present in the image data captured by the cell treatment apparatus.

**12.** The learning apparatus according to any one of claims 6 to 11, further comprising a validation section, wherein the validation section is configured to validate a precision of the learned model based on the obtained learned model and the training data.

**13.** The learning apparatus according to any one of claims 6 to 12, further comprising a storage section, wherein the storage section is configured to store the learned model.

**14.** The learning apparatus according to any one of claims 6 to 13, capable of bidirectionally communicating with a plurality of the cell treatment apparatuses.

**15.** A proposal apparatus for proposing a learned model used for detection of a target cell, the proposal apparatus comprising:

a storage section;
a receiving section;
an acquisition section;
a detection section;
a precision examination section; and
a proposal output section, wherein
the storage section stores a plurality of learned models generated through machine learning using a set of training data composed of: image data that comprises a target cell; and image data that specifies a region where the target cell is present in the image data,
the receiving section receives image data that comprises a cell,
the acquisition section acquires image data that specifies a region where the target cell is present in the image data received by the receiving section,
the detection section detects, using the image data received by the receiving section and the respective learned models stored in the storage section, the target cell in the image data,
the precision examination section examines a precision of each learned model by comparing the region where the target cell is present, detected using each learned model and the image data that specifies the region where the target cell is present, acquired by the acquisition section, and
when there is a learned model satisfying a predetermined precision level, the proposal output section outputs proposal information that comprises information on the learned model satisfying the predetermined precision level.

**16.** The proposal apparatus according to claim 15, wherein, when there is no learned model satisfying the predetermined precision level, the proposal output section outputs proposal information that proposes generation of a new learned model.

**17.** The proposal apparatus according to claim 15 or 16, further comprising a model extraction section, wherein

each learned model is associated with an image capturing condition of the image data that comprises the target cell and is used in the generation of the learned model and/or a culture condition of the cell,
the image data received by the receiving section is associated with the image capturing condition of the image data and/or the culture condition of the cell,
the model extraction section extracts a learned model suitable for the image data received by the receiving section based on the image capturing condition and/or the culture condition associated with the image data

received by the receiving section and the image capturing condition and/or the culture condition associated with each learned model, and
the detection section detects, using the image data received by the receiving section and the extracted learned model, the target cell in the image data.

**18.** A cell treatment method performed by a cell treatment apparatus used for cell treatment,

the cell treatment apparatus comprising:

an observation unit; and
a laser irradiation unit, wherein
the observation unit is capable of capturing an image of a cell that is in a cell culture tool, and
the laser irradiation unit is capable of applying a laser to an inside of the cell culture tool,

the cell treatment method comprising:

a detection step;
an irradiation region setting step; and
a laser irradiation control step, wherein
in the detection step, using image data that comprises the cell captured by the observation unit and a learned model capable of detecting a target cell or a non-target cell, a target cell or a non-target cell in the image data is detected,
in the irradiation region setting step, a region where the target cell is present or a region where the non-target cell is not present is set as a laser irradiation region to be subjected to laser irradiation by the laser irradiation unit, and
in the laser irradiation control step, a laser emitted from the laser irradiation unit is applied to the laser irradiation region in the cell culture tool to treat the target cell.

**19.** The cell treatment method according to claim 18, further comprising an irradiation region adjustment step, wherein

in the irradiation region adjustment step,
a position of a boundary of the laser irradiation region is adjusted based on region adjustment information set in advance,
the region adjustment information is information for adjusting the position of the boundary of the laser irradiation region to reduce or enlarge the laser irradiation region, and
in the laser irradiation control step, a laser emitted from the laser irradiation unit is applied to the thus-adjusted laser irradiation region in the cell culture tool to treat the target cell.

**20.** The cell treatment method according to claim 19, wherein

the region adjustment information comprises information that specifies whether the laser irradiation region should be enlarged or reduced and information that specifies a distance by which the boundary of the laser irradiation region is moved in a normal direction to the boundary, and
in the irradiation region adjustment step, the position of the boundary of the laser irradiation region is moved in the normal direction by the specified distance based on the region adjustment information, whereby the laser irradiation region is enlarged or reduced.

**21.** The cell treatment method according to any one of claims 18 to 20, wherein the learned model is generated through machine learning using a set of training data composed of: image data that comprises a target cell; and image data that specifies a region where the target cell is present in the image data.

**22.** The cell treatment method according to any one of claims 18 to 21, wherein the treatment is to kill the cell.

**23.** A learning method performed by a learning apparatus, the learning method comprising:

a learning step, wherein
in the learning step, a learned model for detecting a target cell in image data that comprises a cell is generated through machine learning using a set of training data composed of: image data captured by the cell treatment

apparatus; and image data that specifies a region where the target cell is present in the image data.

**24.** The learning method according to claim 23, further comprising a receiving step, wherein in the receiving step, the image data captured by the cell treatment apparatus is received.

**25.** The learning method according to claim 23 or 24, wherein

the image data captured by the cell treatment apparatus is associated with an image capturing condition of the image date captured by the cell treatment apparatus and/or a culture condition of the cell, and
in the learning step, the generated learned model is associated with the image capturing condition and/or the culture condition.

**26.** The learning method according to claim 25, wherein the culture condition is at least one condition selected from the group consisting of a cell line name, a cell type, the number of cell passages, a cell seeding density at the start of culture, a culture medium, the number of days used for cell culture, a type of a cell culture vessel, a type of an extracellular matrix, an operator's name, an operator's qualification, and the number of years of operator's work experience.

**27.** The learning method according to claim 25 or 26, wherein the image capturing condition is at least one condition selected from the group consisting of a type of an image sensor, a sensitivity of the image sensor, an exposure time, an aperture value, a lens magnification, a type of a light source, a quantity of light, an illumination time, and an observation method.

**28.** The learning method according to any one of claims 23 to 27, further comprising an acquisition step, wherein in the acquisition step, the image data that specifies the region where the target cell is present in the image data captured by the cell treatment apparatus is acquired.

**29.** The learning method according to any one of claims 23 to 28, further comprising a validation step, wherein in the validation step, based on the obtained learned model and the training data, a precision of the learned model is validated.

**30.** The learning method according to any one of claims 23 to 29, further comprising a storing step, wherein in the storing step, the learned model is stored.

**31.** The learning method according to any one of claims 23 to 30, wherein a plurality of the cell treatment apparatuses are used.

**32.** A learned-model proposal method performed by a proposal apparatus for proposing a learned model used for detection of a target cell, the proposal method comprising:

a receiving step;
an acquisition step;
a detection step;
a precision examination step; and
a proposal output step, wherein
in the receiving step, image data that comprises a cell is received,
in the acquisition step, image data that specifies a region where the target cell is present in the image data received in the receiving step is acquired,
in the detection step, using the image data received in the receiving step and each learned model stored in a storage section, the target cell in the image data is detected,
the storage section stores a plurality of learned models generated through machine learning using a set of training data composed of: the image data that comprises the target cell; and the image data that specifies the region where the target cell is present in the image data,
in the precision examination step, a precision of each learned model is examined by comparing the region where the target cell is present, detected using each learned model stored in the storage section and the image data that specifies the region where the target cell is present, acquired in the acquisition step, and
in the proposal output step, when there is a learned model satisfying a predetermined precision level, proposal information that comprises information on the learned model satisfying the predetermined precision level is

outputted.

**33.** The proposal method according to claim 32, wherein, in the proposal output step, when there is no learned model satisfying the predetermined precision level, proposal information that proposes generation of a new learned model is outputted.

**34.** The proposal method according to claim 32 or 33, further comprising a model extraction step, wherein

each learned model is associated with an image capturing condition of the image data that comprises the target cell and is used in the generation of the learned model and/or a culture condition of the cell,
the image data received in the receiving step is associated with the image capturing condition of the image data and/or the culture condition of the cell,
in the model extraction step, a learned model suitable for the image data received in the receiving step is extracted based on the image capturing condition and/or the culture condition associated with the image data received in the receiving step and the image capturing condition and/or the culture condition associated with each learned model, and
in the detection step, using the image data received in the receiving step and the extracted learned model, the target cell in the image data is detected.

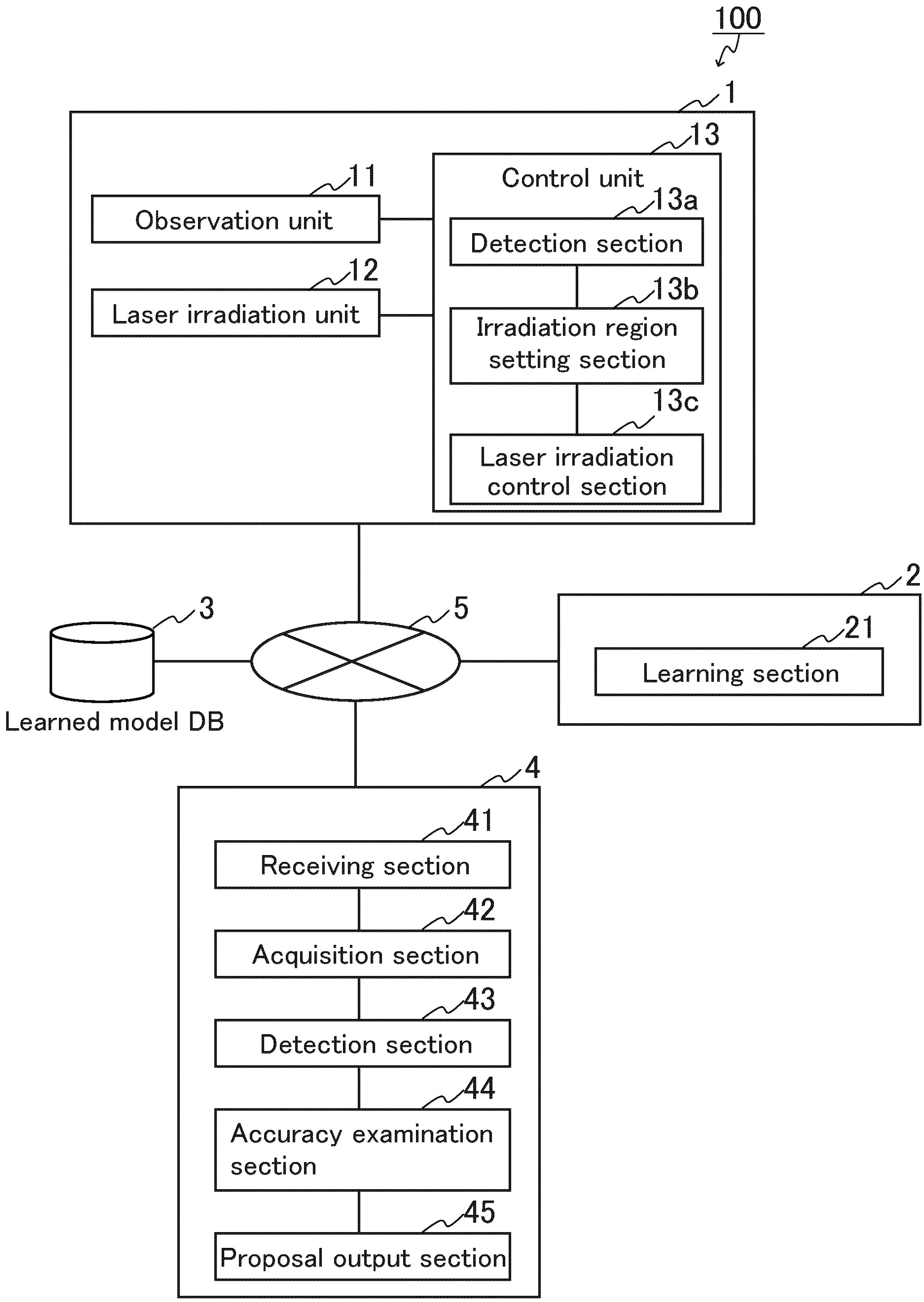
100
1
13
11
Control unit
13a
Observation unit
Detection section
12
13b
Laser irradiation unit
Irradiation region setting section
13c
Laser irradiation control section
2
3
5
21
Learning section
Learned model DB
4
41
Receiving section
42
Acquisition section
43
Detection section
44
Accuracy examination section
45
Proposal output section

FIG. 1

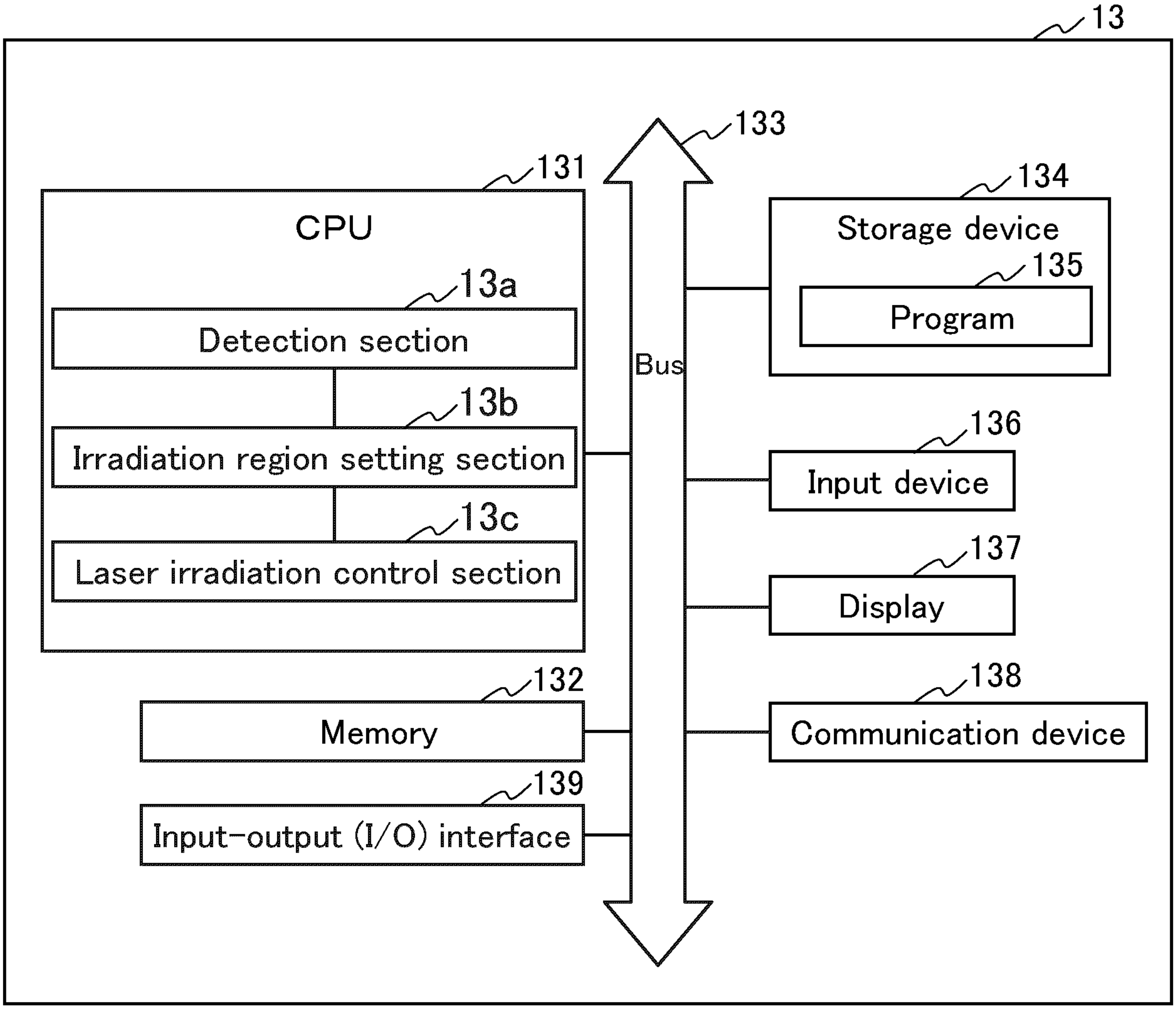
13
133
131
CPU
13a
Detection section
13b
Irradiation region setting section
13c
Laser irradiation control section
Bus
134
Storage device
135
Program
136
Input device
137
Display
138
Communication device
132
Memory
139
Input-output (I/O) interface

FIG. 2

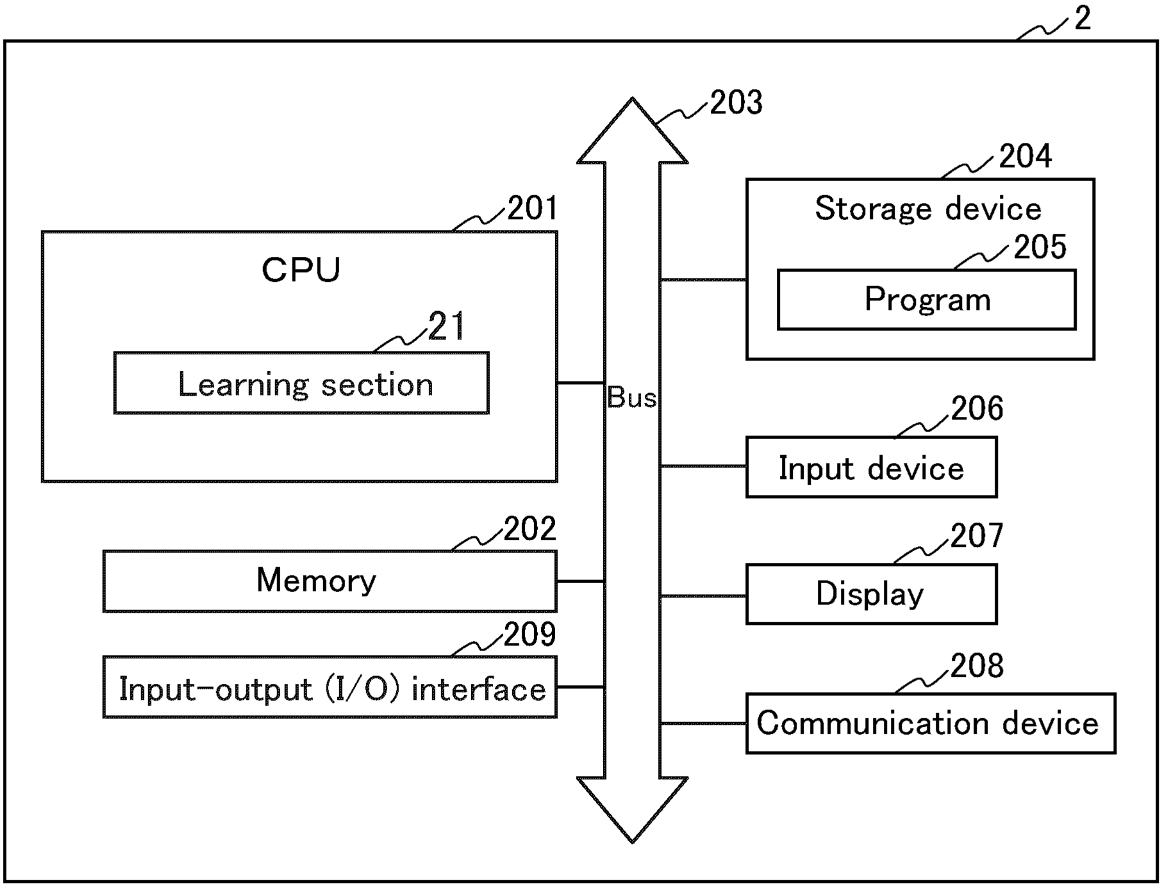
2
203
204
Storage device
205
Program
201
CPU
21
Learning section
Bus
206
Input device
202
Memory
207
Display
209
Input-output (I/O) interface
208
Communication device

FIG. 3

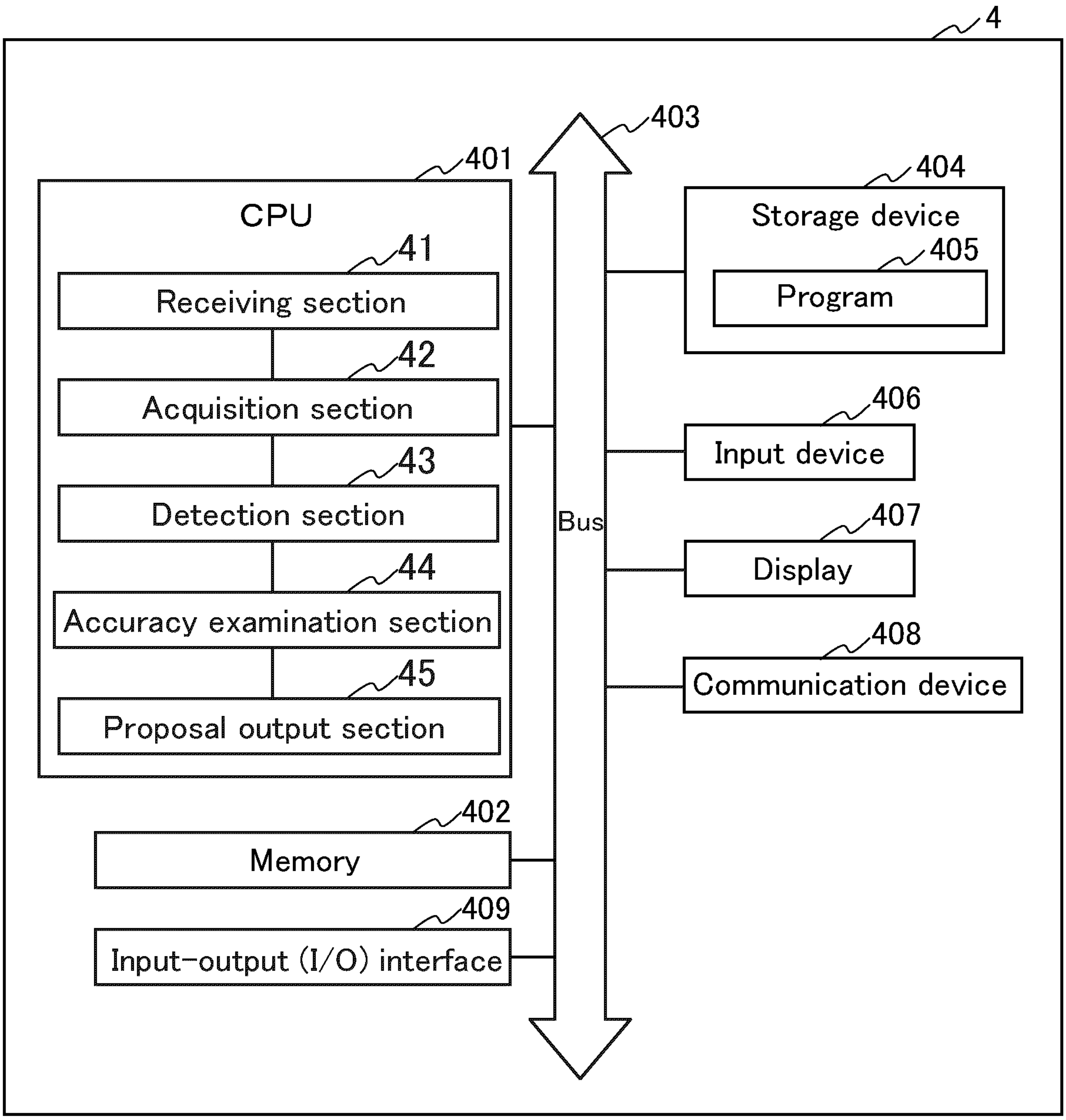
4
403
401
CPU
41
Receiving section
42
Acquisition section
43
Detection section
44
Accuracy examination section
45
Proposal output section
402
Memory
409
Input-output (I/O) interface
Bus
404
Storage device
405
Program
406
Input device
407
Display
408
Communication device

FIG. 4

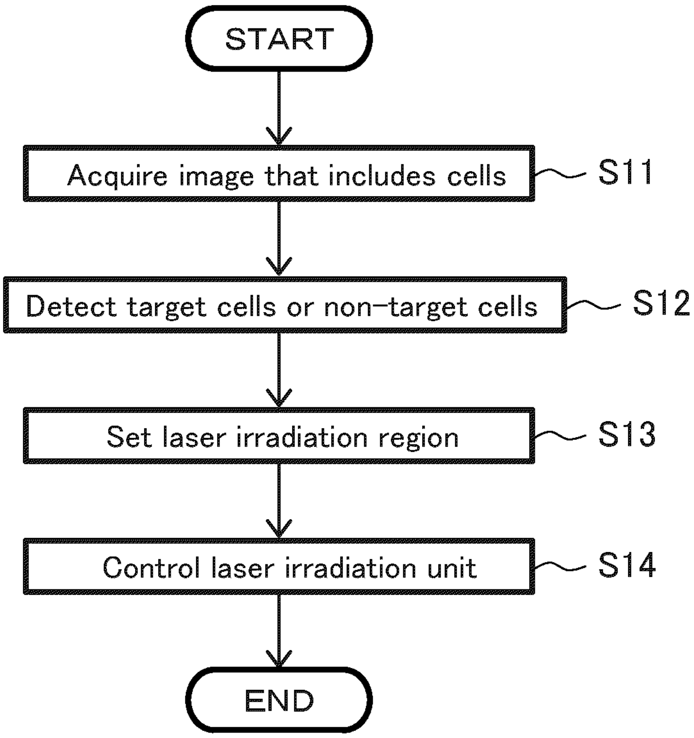
START
Acquire image that includes cells
S11
Detect target cells or non-target cells
S12
Set laser irradiation region
S13
Control laser irradiation unit
S14
END

FIG. 5

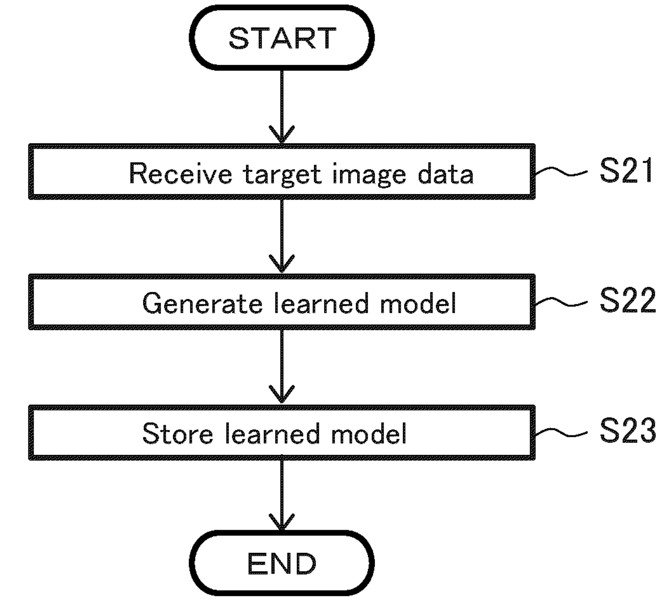
START
Receive target image data
S21
Generate learned model
S22
Store learned model
S23
END

FIG. 6

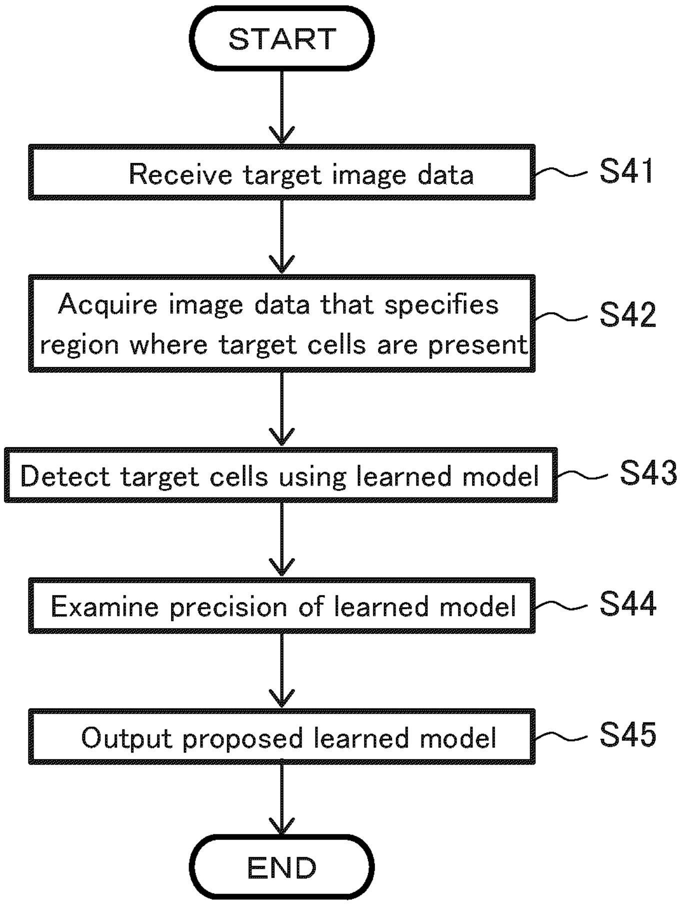
START
Receive target image data
S41
Acquire image data that specifies region where target cells are present
S42
Detect target cells using learned model
S43
Examine precision of learned model
S44
Output proposed learned model
S45
END

FIG. 7

## INTERNATIONAL SEARCH REPORT

International application No.
PCT/JP2021/019472

A. CLASSIFICATION OF SUBJECT MATTER
Int.Cl. C12M1/34(2006.01)i, G06T7/00(2017.01)i, C12Q1/04(2006.01)i
FI: C12M1/34A, C12Q1/04, G06T7/00630

According to International Patent Classification (IPC) or to both national classification and IPC

B. FIELDS SEARCHED
Minimum documentation searched (classification system followed by classification symbols)
Int.Cl. C12M1/34, G06T7/00, C12Q1/04

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X | JP 2017-212944 A (KATAOKA SEISAKUSHO KK) 07 December 2017 (2017-12-07), claim 1, paragraphs [0051], [0065] | 1-5, 18-22 |
| X<br>Y | WO 2019/230447 A1 (FRONTIER PHARMA INC.) 05 December 2019 (2019-12-05), claim 10 | 6-11, 13, 14, 23-28, 30, 31<br>12, 15-17, 29, 32-34 |
| Y | WO 2020/090947 A1 (MIRACA RES INST GK) 07 May 2020 (2020-05-07), paragraph [0079] | 12, 15-17, 29, 32-34 |
| Y | WO 2020/116115 A1 (HOYA CORPORATION) 11 June 2020 (2020-06-11), paragraph [0101] | 12, 15-17, 29, 32-34 |
| Y | WO 2019/229789 A1 (OPTIM CORPORATION) 05 December 2019 (2019-12-05), abstract, claims | 15-17, 32-34 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 July 2021 | 03 August 2021 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2021/019472

**Box No. II Observations where certain claims were found unsearchable (Continuation of item 2 of first sheet)**

This international search report has not been established in respect of certain claims under Article 17 (2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**

International application No. PCT/JP2021/019472

**Box No. III Observations where unity of invention is lacking (Continuation of item 3 of first sheet)**

This International Searching Authority found multiple inventions in this international application, as follows:

(Invention 1) Claims 1-14 and 18-31

Claims 1-11, 13, 14, 18-28, 30, and 31 lack novelty in light of document 1 or 2, and thus do not have a special technical feature. However, claim 12 that depends from claim 1 has the special technical feature of verifying the accuracy of a trained model on the basis of the trained model and teacher data. Claim 29 has the same technical feature as claim 12.

Accordingly claims 1-14 and 18-31 are classified as invention 1.

(Invention 2) Claims 15-17 and 32-34

Claims 15-17 and 32-34 cannot be said to have the same or corresponding technical features between these claims and claim 12 classified as invention 1.

Furthermore, claims 15-17 and 32-34 are not substantially identical to or similarly closely related to any of the claims classified as invention 1.

Accordingly claims 15-17 and 32-34 cannot be identified as invention 1.

Meanwhile, claims 15-17 and 32-34 have the special technical feature of storing multiple trained models, and outputting proposal information including information on a trained model that satisfies predetermined accuracy; thus these claims are classified as invention 2.

Form PCT/ISA/210 (continuation of first sheet) (January 2015)

**INTERNATIONAL SEARCH REPORT**
Information on patent family members

International application No.
PCT/JP2021/019472

| | | |
|---|---|---|
| JP 2017-212944 A | 07 December 2017 | WO 2017/208589 A1<br>EP 3467091 A1<br>claim 1, paragraphs [0050], [0062]<br>TW 201742916 A |
| WO 2019/230447 A1 | 05 December 2019 | (Family: none) |
| WO 2020/090947 A1 | 07 May 2020 | CN 112889088 A |
| WO 2020/116115 A1 | 11 June 2020 | (Family: none) |
| WO 2019/229789 A1 | 05 December 2019 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014509192 A
- WO 2020071332 A
- JP 2020107143 A